# EUROPEAN PATENT APPLICATION

(11) **EP 3 431 110 A1**
(43) Date of publication of application: **23.01.2019**
(21) Application number: 17182906.2
(22) Date of filing: 25.07.2017
(51) Int. Cl.: A61L 9/03

(54) **REPLACEABLE MULTILAYER BREATHING FILM-BASED AROMA CAPSULE AND AROMA-DIFFUSING HEATING DEVICE USING SAME**

(30) Priority: 18.07.2017 US 201715653362
(71) Applicant: Hsiao, Ming Jen, Toufen, Miaoli County 35147 (TW)
(72) Inventor: Hsiao, Ming Jen, Toufen, Miaoli County 35147 (TW)
(74) Representative: Viering, Jentschura & Partner mbB Patent- und Rechtsanwälte

(57) **Abstract**

An aroma-diffusing heating device (1) includes a heating base (10), and a aroma capsule (12) put in the heating base (10) in a replaceable manner and including a heat-transfer container (120), an aromatic substance (122) placed in the heat-transfer container (120), a breathing film (126) consisting of a fiber fixation layer (1261) with first pores (1263) and a microporous layer (1262) with second pores (1264) and bonded to the heat-transfer container (120) and a sealing cover releasably bonded to the heat-transfer container (120) over the breathing film (126).

## Description

### BACKGROUND OF THE INVENTION

### 1. Field of the Invention:

The present invention relates to scent releasing devices and more specifically, to a multilayer breathing film-based aroma capsule and an aroma-diffusing heating device using the replaceable multilayer breathing film-based aroma capsule.

### 2. Description of the Related Art:

In application of the aroma-diffusing heating device designs of US Pat. No. 8,668,885, US Pat. No. 9,550,358, US Pat. No. 9,498,553 and EP Pat. No. 2679249 that are invented by the present inventor, when the heat-transfer container is heated, the heat energy is transferred to the breathing film. Since the breathing film is a plastic film having porous therein, it can be deformed after a long use, causing wrinkles or even affecting the flowing efficiency of the generated aromatic vapor molecules therethrough toward the outside open air. Further, it is not easy to bond the plastic breathing film to the heat-transfer container over the top opening.

Further, other conventional aroma-diffusing heating device designs are also not satisfactory in function and have drawbacks. In the use of conventional aroma-diffusing heating device designs, it is inconvenient to refurnish the aromatic substance. The user needs to prepare a specific heat-transfer container for holding the aromatic substance. When in use, the user needs to take the aromatic substance out of the heat-transfer container carefully, and then to put the aromatic substance in the top accommodation open chamber of the aroma-diffusing heating device, avoiding direct contact of the hands with the aromatic substance.

Further, after the aromatic substance in the accommodation chamber of the heat-transfer container is used up, the user needs to clean the heat-transfer container. If the heat-transfer container is not well cleaned after each use, a new supply of aromatic substance will be mixed with the residual aromatic substance in the accommodation chamber, giving off a bad smell. Further, if the heat-transfer container is made of a fragile material (for example, ceramics or glass), it may be forced down or broken easily during cleaning, causing the aromatic substance to fall to the ground or to the fluid aromatic substance to contaminate the aroma-diffusing heating device or the surroundings.

Therefore, it is desirable to provide an aroma-diffusing design that eliminates the drawbacks of the aforesaid prior art designs.

### SUMMARY OF THE INVENTION

The present invention has been accomplished under the circumstances in view. It is therefore an object of the present invention to provide a multilayer breathing film-based aroma capsule and an aroma-diffusing heating device using the multilayer breathing film-based aroma capsule that eliminate the drawbacks of the conventional aroma-diffusing heating device designs.

The invention has the advantages as follows:
In the operation of the aroma-diffusing heating device to heat the aroma capsule, the breathing film is constantly kept in shape without wrinkles, allowing generated aromatic vapor molecules to smoothly flow through the multilayer breathing film to the outside open air.

The multilayer breathing film effectively prohibits the fluid aromatic substance from flowing out of the top opening of the aroma capsule to contaminate the aroma-diffusing heating device or the surroundings if the aroma capsule or the aroma-diffusing heating device falls down accidentally during the heating operation.

After the aromatic substance of the aroma capsule is used up, the aroma capsule can be recycles, and a new aroma capsule can be placed in the aroma-diffusing heating device conveniently, facilitating the application.

The aroma-diffusing heating device comprises a heating base defining a top accommodation open chamber, an aroma capsule placed in the top accommodation open chamber of the heating base, and a cover member fastened to the heating base to hold the aroma capsule in the heating base.

In one embodiment of the present invention, the aroma capsule comprises a heat-transfer container defining a top opening, an aromatic substance placed in the heat-transfer container, and a breathing film having a breathable function and bonded to the heat-transfer container over the top opening to hold the aromatic substance in the heat-transfer container. When in use, the aroma capsule is placed in the top accommodation open chamber of the heating base, allowing the aromatic substance to be heated to release aromatic vapor molecules,

In one embodiment of the present invention, the breathing film comprises a fiber fixation layer defining therein a plurality of first pores, and a microporous layer defining therein a plurality of second pores. The fiber fixation layer is bonded with the microporous layer to create the breathing film that has a breathable function. The microporous layer can be made out of thermoplastic elastomer (TPE). The thermoplastic elastomer (TPE) microporous layer has the second pores therein. The fiber fixation layer is bonded with the thermoplastic elastomer (TPE) microporous layer to create the breathing film. The fiber fixation layer has the characteristics of anti-deformation, good weather resistance and high dimensional stability, and can effectively keep the microporous layer in shape without wrinkles during the heating process, maintaining the breathing function.

The base fiber material of the fiber fixation layer can be organic or inorganic fibers, such as polymeric fibers, chemical fibers, polyester, cellulose, rayon, glass fibers, and carbon fibers. Alternatively, natural fibers such as plant fibers, wood fibers, silk, and paper fibers can be selectively used. The base fiber material is a porous thin film, fabric or nonwoven fabric in a predetermined shape and size, having a certain degree of heat resistance.

During the fabrication of the fiber fixation layer, the aperture of the first pores of the fiber fixation layer can be controlled by means of the control of the fiber tightness of the matrix material. Pore forming agent can also be used in the fabrication of the fiber fixation layer to form the desired first pores.

The microporous layer has a waterproof and moisture permeable function. The microporous layer is a multi-(micro) hole matrix material, or a microporous film or fabric made of a matrix material (such as polymer) with the application of a hole forming agent (gas or filler). The microporous layer defines therein a plurality of second pores. During the fabrication of the microporous layer, multiple micro pores are formed therein subject to the use of a hole forming agent. McCormark WO 96/19346 discloses methods of making breathable microporous films having zoned breathability.

In some embodiments of the present invention, the microporous layer is selected from, but not limited to, films made using, for example, thermoplastic elastomer (TPE) compositions, which are prepared by melt-plasticizing the film, or by, for example, stretching the film to create micro pores. Filler particles or hole forming agent can be selectively used for making the microporous layer to create the desires micro holes. The applied filler material, oil or hole forming agent is mixed with the thermoplastic elastomer (TPE). Forming, solution film forming, stretching, electrostatic spinning and direct drilling techniques can be selectively used for making a thin film with µm grade micro pores of size even below 10µm. Precision instrument or etching technique can be employed for the creation of the desired micro pores. The use of a thermoplastic elastomer (TPE) film without pores can simply selectively allow gas to pass. The open space of each of the second pores allows the released aromatic vapor molecules to pass therethrough. The choice of microporous layer with moisture permeability and good masking properties can block melted aromatic substance or spices fluid, prohibiting melted aroma wax or essential oil from passing therethrough. The second pores of the microporous layer provide waterproof and moisture permeable effects. The microporous layer and the fiber fixation layer are bonded together to create the desired breathing film that is then bonded to the heat-transfer container over the top opening to block the melted aromatic substance from falling to the outside of the aroma capsule.

In one embodiment of the present invention, the thermoplastic elastomer (TPE) microporous layer defines therein a plurality of second pores. Preferably, the thermoplastic elastomer (TPE) is thermoplastic urethane (TPU); the fiber fixation layer is TETORON or polyethylene terephthalate (PET). TETORON is formed by the condensation of terephthalic acid and ethylene glycol linear polymer. The fabric made out of polyethylene terephthalate is called as Dacron that is hot pressed to create the fiber fixation layer. Glass fiber can be added to polyethylene terephthalate to enhance stiffness and heat resistance with the first pores defined therein. Polyester products such as polyester fabric or nonwoven fabric can be used as a substitute. Silk cloth can also be selectively used for the fiber fixation layer.

In one embodiment of the present invention, the heat-transfer container is selected from the material group consisting of polymers, metal, ceramic and wood. The polymers can be plastic, polyester plastic, PCTG, TPU, PET or PP.

In one embodiment of the present invention, the heat-transfer container further comprises a rim extended around the top opening thereof; the breathing film is bonded to the rim of the heat-transfer container.

In one embodiment of the present invention, the thermoplastic elastomer (TPE) microporous layer of the breathing film is bonded to the heat-transfer container over the top opening.

In one embodiment of the present invention, the heat-transfer container is made out of polymers, for example, the heat-transfer container is made out of poly chloro terephthalate glycol (PCTG). The thermoplastic elastomer microporous layer is made out of thermoplastic urethane (TPU) to create the breathing film. The TPU material of the breathing film is bonded to the PCTG heat-transfer container over the top opening and the aromatic substance. The use of TETORON or PET fiber fixation layer can be constantly maintained in shape and kept free of the effect of weather. The first pores of the fiber fixation layer are relatively larger than the second pores of the TPU microporous layer. During the operation of the aroma-diffusing heating device to heat the aroma capsule, the TETORON or PET fiber fixation layer does not shrink or expand during the heating process, and effectively keeps the TPU microporous layer in shape without collapsing the second pores, and thus, the breathing film can be maintained in a good looking, and the ventilation of the second pores of the TPU microporous layer is maintained in function. The TPU microporous layer works like the functioning of a GOTEX fabric that prohibits external water molecules from passing through the second pores of the TPU microporous layer into the inside of the aroma capsule to wet the aromatic substance. During the operation of the aroma-diffusing heating device to heat the aromatic substance, a high concentration of aromatic vapor molecules is generated. The aromatic vapor molecules then flow through the second pores of the TPU microporous layer of the breathing film and then the first pores of the TETORON or PET microporous layer to the outside open air.

In one embodiment of the present invention, the size of the first pores of the breathable aperture is greater than or equal to the size of the second pores of the breathable aperture, i.e., the ventilatory capacity per unit area of the first pores of the fiber fixation layer is greater than or equal to the ventilatory capacity per unit area of the second pores of the microporous layer.

In one embodiment of the present invention, the porosity of the first pores and the porosity of the second pores are different.

In one embodiment of the present invention, the PET fiber fixation layer and the TPU microporous layer are bonded together using hot-press fusion or high-frequency sealing technology to create the breathing film. The TPU material of the breathing film is bonded to the rim of the PCTG heat-transfer container over the top opening.

In one embodiment of the present invention, the heat-transfer container further comprises a sealing cover bonded to the top opening over the breathing film and the aromatic substance to seal the aromatic substance in the heat-transfer container, preventing the aromatic substance from being in contact with the atmosphere.

In one embodiment of the present invention, the aroma-diffusing heating device further comprises a cover member fastened to the heating base to hold the aroma capsule in the heating base. The cover member defines therein a hole for the passing of the generated aromatic vapor molecules to the outside open air.

In one embodiment of the present invention, the aromatic substance is selected from the group consisting of aromatic wax, perfume, balsam, sesame oil mixture and essential oil.

The invention achieves the effects as follows:
In the operation of the aroma-diffusing heating device to heat the aroma capsule, the breathing film is maintained in shape, allowing the aromatic vapor molecules released by the aromatic substance to flow smoothly through the breathing film to the outside open air

After the aromatic substance of the aroma capsule is used up, the aroma capsule can be recycled, and another new aroma capsule can be conveniently put in the aroma-diffusing heating device for application.

### BRIEF DESCRIPTION OF THE DRAWINGS

FIG. 1 is an exploded view of an aroma capsule in accordance with the present invention.
FIG. 2 is a cross-sectional view of the aroma capsule in accordance with the present invention.
FIG. 3 is an elevational view of the aroma capsule in accordance with the present invention illustrating the sealing cover removed from the heat-transfer container and the breathing film opened from the heat-transfer container.
FIG. 4 is an exploded view of an aroma-diffusing heating device in accordance with the present invention.
FIG. 5 illustrates the outer appearance of the aroma-diffusing heating device in accordance with the present invention.
FIG. 6 is a sectional view of FIG. 5.
FIG. 7 illustrates the ornamental outer shell and the cover member fastened to the aroma-diffusing heating device.
FIG. 8 is an exploded view of an alternate form of the aroma capsule in accordance with the present invention.

### DETAILED DESCRIPTION OF THE PREFERRED EMBODIMENT

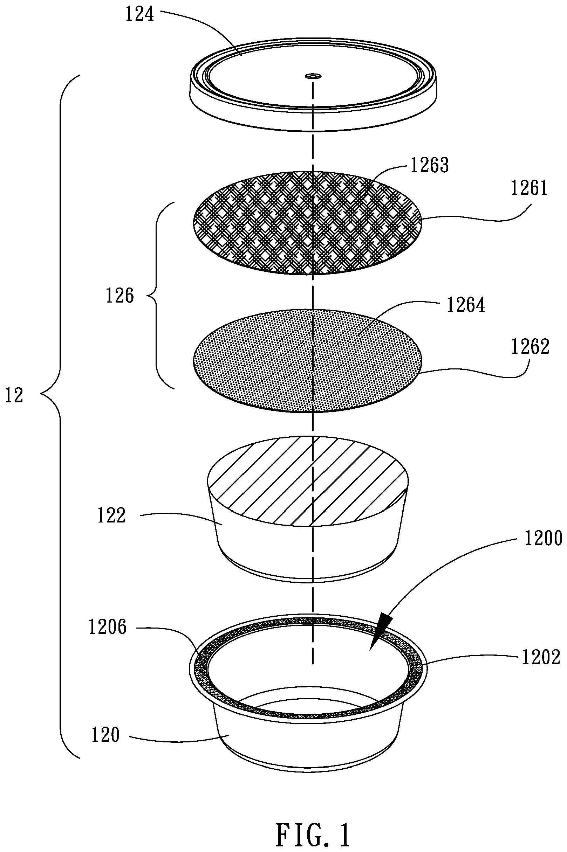

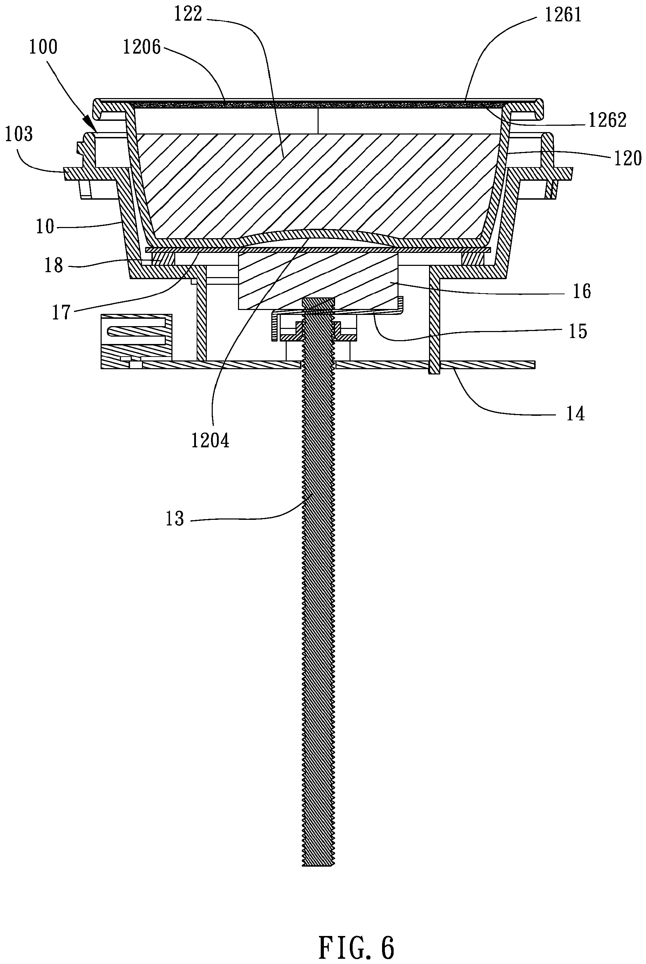

Referring to FIGS. 1-6, an aroma-diffusing heating device **1** in accordance with the present invention is shown. The aroma-diffusing heating device **1** comprises a heating base **10** defining a top accommodation open chamber **100,** and an aroma capsule **12** detachably accommodated in the top accommodation open chamber **100** of the heating base **10** and heatable by the heating base **10** to give off fragrance. The aroma capsule **12** comprises a heat-transfer container **120** defining a top opening **1200,** an aromatic substance **122** held in the heat-transfer container **120,** and a breathing film bonded to the heat-transfer container **120** over the top opening **1200** and the aromatic substance **122** to allow moisture vapor to be transmitted through the material.

Referring to FIGS. 1-6, in one embodiment of the present invention, the breathing film **126** comprises a fiber fixation layer **1261** and a microporous layer **1262** of excellent moisture permeability. The fiber fixation layer **1261** defines therein multiple first pores **1263.** The microporous layer **1262** defines therein multiple second pores **1264.** The fiber fixation layer **1261** and the microporous layer **1262** are bonded together to form the breathing film **126** with the breathable and waterproof function. The microporous layer **1262** is preferably made out of thermoplastic elastomer (TPE) compound. The fiber fixation layer **1261** is preferably made out of polyester. The polyester fiber fixation layer **1261** and the thermoplastic elastomer (TPE) microporous layer **1262** are bonded together to form the breathing film **126** that is breathable. The breathable aperture of the first pores **1263** is greater than the breathable aperture of the second pores **1264,** facilitating outward transmittance of air. The breathing film **126** is bonded to the heat-transfer container **120** over the top opening **1200.** Through the second pores **1264** of the thermoplastic elastomer (TPE) microporous layer **1262,** the aroma capsule **12** provides waterproof and moisture transmissive functions, keeping the aromatic substance **122** fresh.

The thermoplastic elastomer (TPE) microporous layer **1262** and the fiber fixation layer **1261** can be bonded together using hot-press fusion or high-frequency sealing technology. Since the first pores **1263** of the fiber fixation layer **1261** are relatively lager than the second pores **1264** of the thermoplastic elastomer (TPE) microporous layer **1262,** the heated aromatic substance vapor molecules can go through second pores **1264** of the thermoplastic elastomer (TPE) microporous layer **1262** toward the first pores **1263** of the fiber fixation layer **1261.**

In application, the aroma capsule **12** is heated in the aroma-diffusing heating device **1** to release a pleasant smell at a temperature below 90°C. During the heating process, the fiber fixation layer **1261** does not shrink or expand the size and can keep the thermoplastic elastomer (TPE) microporous layer **1262** in shape, and thus, the surface of the breathing film **126** can be constantly maintained smooth without wrinkles. During the heating process, aromatic substance **122** keeps releasing aromatic vapor molecules that flow smoothly through the second pores **1264** of the thermoplastic elastomer (TPE) microporous layer **1262** and the first pores **1263** of the fiber fixation layer **1261** toward the atmosphere outside the aroma capsule **12,** however, the liquid phase aromatic substance is prohibited from passing through the second pores **1264,** ensuring safety application of the aroma capsule **12.**

Further, the aromatic substance **122** is selected from the group consisting of aromatic wax, perfume, balsam, sesame oil mixture and essential oil. In the case an aromatic wax is used for the aromatic substance **122** of the aroma capsule **12,** the heating base **10** simply needs to heat the aromatic wax to the melting point of the aromatic wax, causing the aromatic wax to release aromatic vapor molecules through the breathing film **126** toward the outside open air. In the case a sesame oil mixture or essential oil is used for the aromatic substance **122** of the aroma capsule **12,** the sesame oil mixture or essential oil is heated to release aromatic vapor molecules through the breathing film **126** toward the outside open air. When compared to related existing commercial products, the breathing film **126** of the present invention will not deform during the heating process, and the released aromatic vapor molecules can flow through the breathing film **126** toward the outside open air efficiently.

Further, the heat-transfer container **120** is made out of polymers. In one embodiment, the heat-transfer container **120** is made out of plastics selected from the group consisting of polyester plastic, poly chloro terephthalate glycol (PCTG), polyethylene terephthalate (PET), propylthiouracil (PTU) and polypropylene (PP). The plastic heat-transfer container **120** does not melt or deform at the above-mentioned heating temperature, and can efficiently transfer heat energy from the heating base **10** to the aromatic substance **122.** In one embodiment of the present invention, the heat-transfer container **120** is made out of PCTG, capable of transferring heat energy from the heating base **10** to the aromatic substance **122** for causing the aromatic substance **122** to release a pleasant smell. Further, the heat-transfer container **120** has excellent ductility and toughness and is not easy to rapture. It will not be damaged when heating by the aroma-diffusing heating device, and can efficiently transfer heat energy from the aroma-diffusing heating device to the aromatic substance. When compared to fragile pottery and glass heat-transfer containers of conventional aroma-diffusing heating devices, the heat-transfer container **120** has the advantage that the top opening **1200** of the heat-transfer container **120** can easily be bonded with the breathing film **126,** i.e., the thermoplastic microporous layer **1262** of the breathing film **126** can be bonded to the top opening **1200** of the heat-transfer container **120** by heat or with an adhesive. The bonding effect is better than the prior art design. After bonding, the breathing film **126** will not fall off.

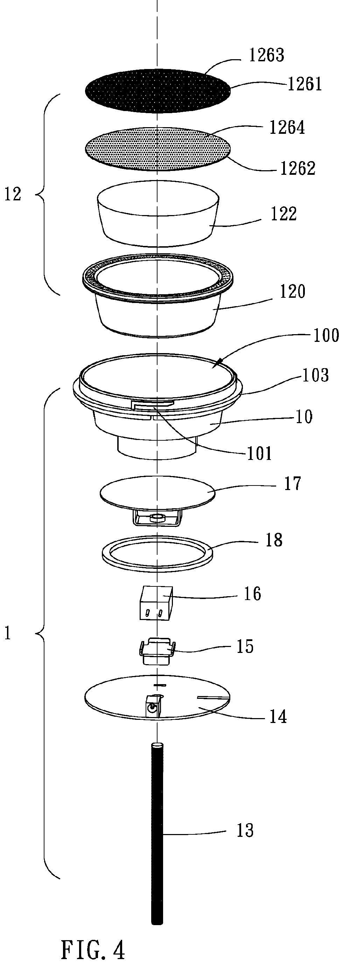

Referring to FIGS. 4, 5 and 6, in application, the user simply needs to put the aroma capsule **12** in the top accommodation open chamber **100,** and then to electrically conduct the heating base **10,** melting the aromatic substance **122** (such as aromatic wax or essential oil) to emit aromatic vapor molecules. In the heating process, the emitted aromatic vapor molecules go from the aroma capsule **12** through the second pores **1264** of the microporous layer **1262** of the breathing film **1261** and then the first pores **1263** of the fiber fixation layer **1261** to the surrounding air while the breathing film **1261** stays in shape and keeps breathing.

After the aromatic substance **122** is used up, the heat-transfer container **120** can be thrown away or recycled, and a new aroma capsule **12** can be put in the top accommodation open chamber **100** of the aroma-diffusing heating device **1.** Thus, the user's hand will not be contaminated by the aromatic substance **122.** Further, the user does not necessary to clean the top accommodation open chamber **100** of the aroma-diffusing heating device **1.** Even if the aroma-diffusing heating device **1** falls down accidentally during application, the melted aromatic substance **122** of the aroma capsule **12** is still held in the heat-transfer container **120** by the breathing film **126** and will not flow to the outside of the aroma-diffusing heating device **1** to cause contamination or dangers.

Further, balsam, spices, sesame oil mixture can be selectively used for the aromatic substance **122** to substitute for aromatic wax. The application of these substances is same as the use of aromatic wax.

Referring to FIGS. 1, 2, 3 and 4, in one embodiment of the present invention, the aroma capsule **12** further comprises a rim **1202** extended around the top opening **1200,** and the breathing film **126** is bonded to the rim **1202.** Preferably, the thermoplastic elastomer (TPE) microporous layer **1262** is peripherally bonded to the rim **1202** of the heat-transfer container **120** over the top opening **1200** to secure the breathing film **126** to the heat-transfer container **120.**

Referring to FIGS. 1, 2, 3 and 4, in one embodiment of the present invention, the heat-transfer container **120** is a plastic heat-transfer container **120**; the fiber fixation layer **1261** is made out of PET; the thermoplastic elastomer (TPE) microporous layer **1262** is made out of thermoplastic polyurethane (TPU); the PET fiber fixation layer **1261** and the thermoplastic elastomer (TPE) microporous layer **1262** are bonded together by hot press bonding or high-frequency sealing without affecting the breathing film **126**; the TPU microporous layer **1262** of the breathing film **126** is peripherally bonded to the top opening **1200** of the plastic (such as PCTG) heat-transfer container **120** by hot press bonding or high-frequency sealing; the TPU microporous layer **1262** is peripherally bonded to the rim **1202** of the PCTG heat-transfer container **120** over the top opening **1200,** and covered over the aromatic substance **122** to seal the aromatic substance **122** in the aroma capsule **12.**

Referring to FIGS. 1, 2, 3 and 4, in one embodiment of the present invention, the plastic heat-transfer container **120** is made out of PCTG; the fiber fixation layer **1261** of the breathing film **126** is made out of PET; the microporous layer **1262** of the breathing film **126** is made out of TPU; subject to the fusible nature between PTU and PCTG, the TPU layer of the breathing film **126** is peripherally bonded to the rim **1202** of the PCTG heat-transfer container **120** around top opening **1200** by an automatic machine, improving the bonding quality and production rate and saving the cost. Further, an adhesive can be used to bond the breathing film **126** and the heat-transfer container **120** together.

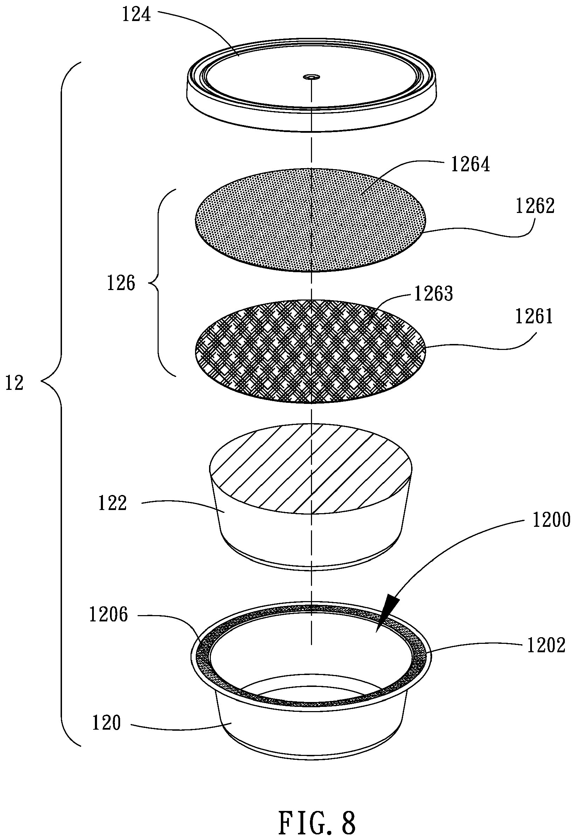

Referring to FIG. 8, the fiber fixation layer **1261** and microporous layer **1262** of the aroma capsule **12** can be swapped upside down. In this case, the top side of the fiber fixation layer **1261** is bonded to the microporous layer **1262** to create the breathing film **126;** the fiber fixation layer **1261** is peripherally bonded to the heat-transfer container **120** over the top opening **1200;** when the aromatic substance **122** (such as aromatic wax, spices or essential oil) is being heated, the emitted aromatic vapor particles go through the first pores **1263** of the fiber fixation layer **1261** and then the second pores **1264** of the microporous layer **1262** toward the outside open air; the effect produced by the breathing film **126** is same is same as described above.

In one embodiment of the present invention, the breathable aperture of the first pores **1263** of the fiber fixation layer **1261** is greater than or equal to the breathable aperture of the second pores **1264** of the microporous layer **1262,** facilitating passing of the generated aromatic vapor molecules through the breathing film **126,** i.e., the ventilatory capacity per unit area of the first pores **1263** of the fiber fixation layer **1261** is greater than or equal to the ventilatory capacity per unit area of the second pores **1264** of the microporous layer **1262,** facilitating passing of released aromatic vapor molecules through the breathing film **126.**

In one embodiment of the present invention, the porosity of the first pores **1263** of the fiber fixation layer **1261** and the porosity of the second pores **1264** of the microporous layer **1262** are unequal.

When the aromatic substance **122** is being heated by the aroma-diffusing heating device **1** to release aromatic vapor molecules, the concentration of gas outside the breathing film **126** of the aroma capsule **12** is lower than the concentration of gas inside the aroma capsule **12,** therefore the high concentration of the aromatic vapor molecules flows through the second pores **1264** of the TPU microporous layer **1262** and the first pores **1263** of the PET fiber fixation layer **1261** toward the outside open air.

Referring to FIGS. 1, 2 and 3, in one embodiment of the present invention, the heat-transfer container **120** further comprises a sealing cover **124** bonded to the top opening **1200** and covered over the breathing film **126** and the aromatic substance **122** to seal the aromatic substance **122** in the heat-transfer container **120.** The sealing cover **124** has an inner protruding portion **1241** protruded from an inner surface thereof and releasably engaging the rim **1202** of the top opening **1200.** In application, the user simply needs to remove the inner protruding portion **1241** of the sealing cover **124** from the rim **1202,** and then to put the aroma capsule **12** in the top accommodation open chamber **100,** and then to electrically conduct the heating base **10** for generating heat to heat the aromatic substance (such as aromatic wax, essential oil or spices) **122** from a solid state into a vapor state, allowing emitted aromatic vapor molecules to flow through the breathing film **126** to the outside open air.

The sealing cover **124** seals the aromatic substance **122** in the heat-transfer container **120,** maintaining the quality of the aromatic substance, preventing the aromatic substance from being in contact with air, and facilitating delivery or storage.

Referring to FIGS. 4, 5, 6 and 7, in one embodiment of the present invention, the aroma-diffusing heating device **1** further comprises a connection unit **13,** a power circuit board **14,** a gasket **15,** a heating element **16,** a heat conduction unit **17,** and a thermal insulator **18.** The power circuit board **14** is affixed to the connection unit **13.** The gasket **15** is supported on one side of the connection unit **13.** The connection unit **13** has an opposite side thereof fastened to an ornamental outer shell **22.** The heating element **16** is mounted on the gasket **15** and disposed in contact with a bottom surface of the heat conduction unit **17;** the heating base **10** defines therein the aforesaid top accommodation open chamber **100;** the heat conduction unit **17** is mounted in a bottom side of the top accommodation open chamber **100,** having one side thereof disposed in contact with the heating element **16** and an opposite side thereof disposed in contact with the aroma capsule **12;** the heating element **16** is electrically connected to the power contacts of the power circuit board **14** for generating heat; the thermal insulator **18** is mounted between the heat conduction unit **17** and the heating base **10** to protect the heating base **10** against thermal damage; the heat conduction unit **17** can be made of metal, ceramic or glass; the heating element **16** can be a positive temperature coefficient (PTC), cement resistor or resistor type heating element; the thermal insulator **18** can be selected from the group consisting of silicon rubber, rubber, plastics and wood.

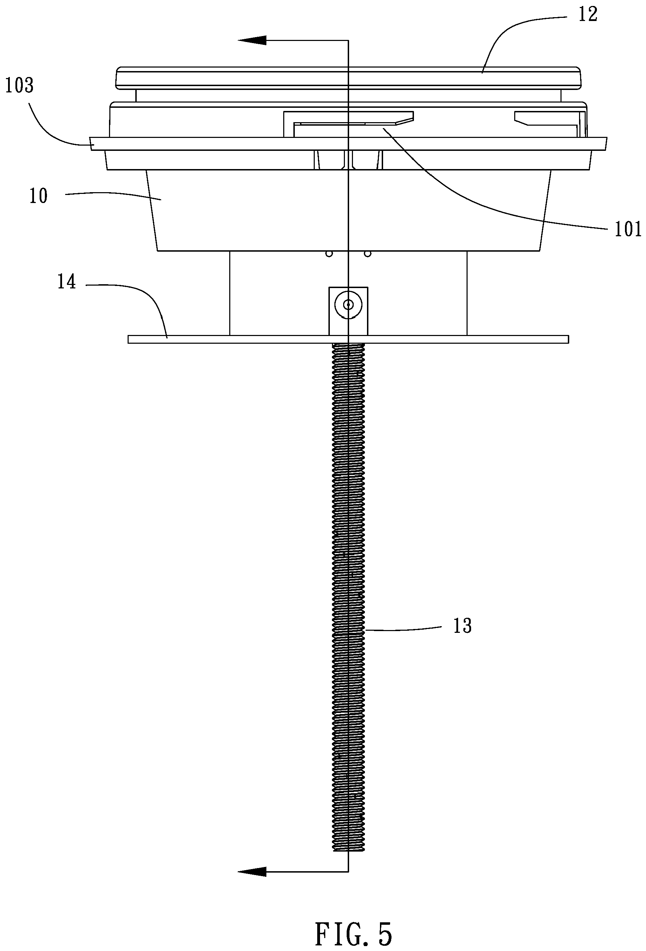

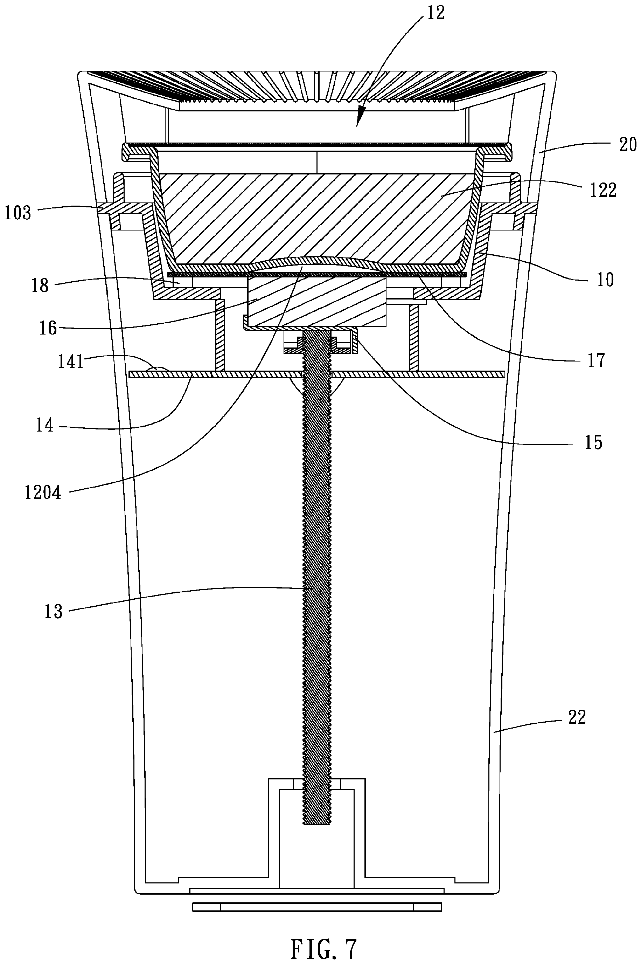

Referring to FIG. 7, in one embodiment of the present invention, the aroma-diffusing heating device **1** further comprises a cover member **20;** the heating base **10** of the aroma-diffusing heating device **1** comprises a retaining groove **101** located on the periphery thereof (see FIG. 5); the cover member **20** comprises a retaining flange protruded from an inner surface thereof and detachably fastened to the retaining groove **101** to secure the cover member **20** to the heating base **10** over the aroma capsule **12,** and a top opening **201** for guiding out emitted aromatic vapor molecules and providing enhanced decorative sense.

Referring to FIG. 7, in one embodiment of the present invention, the heating base **10** is made of a light transmissive or translucent material, such as plastics, glass or silicon rubber; the light transmissive or translucent heating base **10** further comprises a light-guiding rim **103** extended around the periphery thereof and disposed outside the ornamental outer shell **22.** In this embodiment, the light-guiding rim **103** is set between the cover member **20** and the ornamental outer shell **22;** the power circuit board (PCB) **14** further comprises a light-emitting device **141.** The light-emitting device **141** can be, for example, light-emitting diode (LED) or lamp bulb. The power circuit board **14** connects external power source to the light-emitting device **141,** causing the light-emitting device **141** to emit light through the heating base **10** to the light-guiding rim **103** to provide a visual decorative effect.

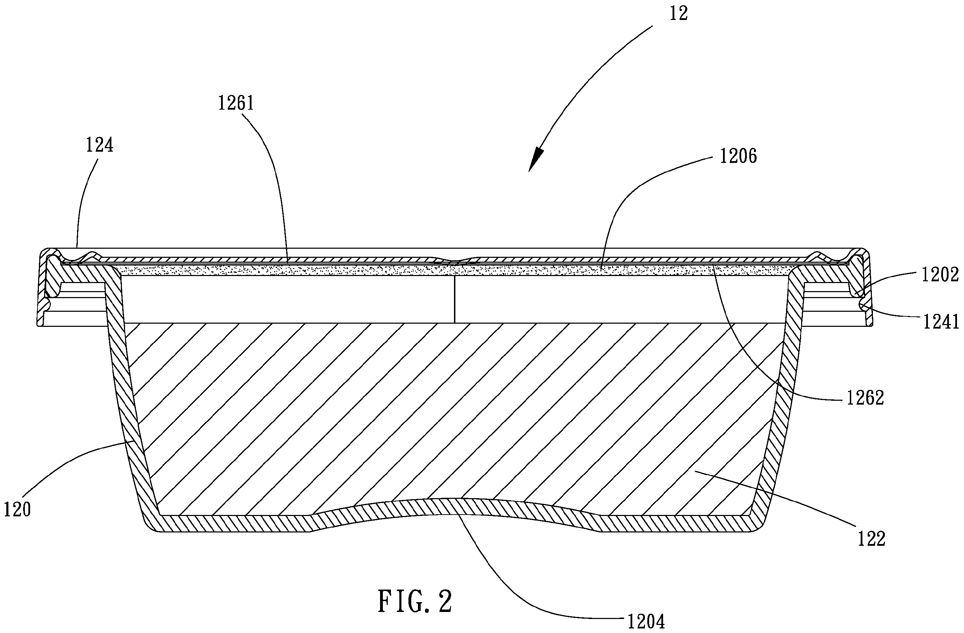

Referring to FIGS. 2, 6 and 7, in one embodiment of the present invention, the heat-transfer container **120** of the aroma capsule **12** has a bottom recess **1204** located on a bottom surface thereof and curving upwards. The high temperature of the central heat source of the heating element **16** is transferred to the center area of the heat conduction unit **17** to heat the center area of the aroma capsule **12** and the aromatic substance **122,** melting the aromatic substance rapidly; the bottom recess **1204** is kept apart from the high temperature of the central heat source of the heating element **16,** enabling the other part of the heat conduction unit **17** beyond the center area to quickly and evenly heat the aroma capsule **12** and the heat-transfer container **120** beyond the bottom recess **1204,** avoiding the aromatic substance **122** from being heated up too fast.

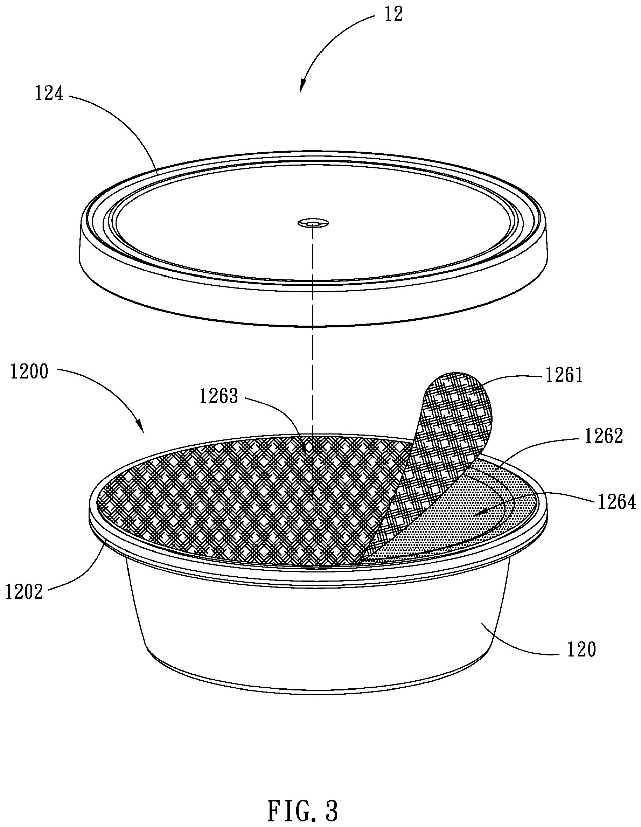

Referring to FIGS. 1 and 3, in one embodiment of the present invention, the rim **1202** of the heat-transfer container **120** of the aroma capsule **12** around the top opening **1200** has a relatively higher coefficient of friction surface **1206,** forming a rough surface so that the breathing film **126** can be bonded to the rim **1202** over the top opening **1200** positively and will not drop off therefrom easily.

Although particular embodiment of the invention have been described in detail for purposes of illustration, various modifications and enhancements may be made without departing from the spirit and scope of the invention. Accordingly, the invention is not to be limited except as by the appended claims.

## Claims

1. An aroma-diffusing heating device (1), comprising:
a heating base (10) defining a top accommodation open chamber (100); and
an aroma capsule (12) put in said top accommodation open chamber (100) of said heating base (10) and heatable to release aromatic vapor molecules, said aroma capsule (12) comprising a heat-transfer container (120) defining a top opening (1200), an aromatic substance (122) put in said heat-transfer container (120) and a breathing film (126) having a breathable function and bonded to said heat-transfer container (120) over said top opening (1200) to hold said aromatic substance (122) in said heat-transfer container (120), said breathing film (126) comprising a fiber fixation layer (1261) defining therein a plurality of first pores (1263) and a microporous layer (1262) bonded to said fiber fixation layer (1261) in a stack and defining therein a plurality of second pores (1264).

2. The aroma-diffusing heating device (1) as claimed in claim 1, wherein said microporous layer (1262) is made out of thermoplastic elastomer with said second pores (1264) defined therein; said fiber fixation layer (1261) is made out of polyester with said first pores (1263) defined therein.

3. The aroma-diffusing heating device (1) as claimed in claim 2, wherein said polyester is selected from the group consisting of Tetoron and polyethylene terephthalate (PET); said thermoplastic elastomer is thermoplastic polyurethane (TPU).

4. The aroma-diffusing heating device (1) as claimed in claim 1, wherein the breathable aperture of said first pores (1263) is greater than the breathable aperture of said second pores (1264).

5. The aroma-diffusing heating device (1) as claimed in claim 1, wherein the porosity of said first pores (1263) and the porosity of said second pores (1264) are different.

6. The aroma-diffusing heating device (1) as claimed in claim 1, wherein said aroma-diffusing heating device (1) further comprises an ornamental outer shell (22), a connection unit (13) connected to said ornamental outer shell (22), a power circuit board (14), a gasket (15), a heating element (16), a heat conduction unit (17) and a thermal insulator (18), said power circuit board (14) being fixedly fastened to said connection unit (13), said connection unit (13) being connected to a bottom side of said heat conduction unit (17), said gasket (15) being supported on said connection unit (13), said heating element (16) being mounted on said gasket (15) and abutted at a bottom side of said heat conduction unit (17), said heat conduction unit (17) being mounted in a bottom side of said top accommodation open chamber (100) and having one side thereof disposed in contact with said heating element (16) and an opposite side thereof disposed in contact with aroma capsule (12), said heating element (16) being electrically connected to power contacts of said power circuit board (14).

7. A multilayer breathing film-based aroma capsule (12), comprising:
a heat-transfer container (120) defining a top opening (1200);
an aromatic substance (122) placed in said heat-transfer container (120); and
a breathing film (126) having a breathable function and being bonded to said heat-transfer container (120) over said top opening (1200) to keep said aromatic substance (122) in said heat-transfer container (120), said breathing film (126) comprising a fiber fixation layer (1261) defining therein a plurality of first pores (1263) and microporous layer (1262) defining therein a plurality of second pores (1264) and bonded to said microporous layer (1262).

8. The multilayer breathing film-based aroma capsule (12) as claimed in claim 7, wherein said microporous layer (1262) is made out of thermoplastic elastomer with said second pores (1264) defined therein; said fiber fixation layer (1261) is made out of polyester with said first pores (1263) defined therein.

9. The multilayer breathing film-based aroma capsule (12) as claimed in claim 8, wherein said polyester is selected from the group consisting of Tetoron and polyethylene terephthalate (PET); said thermoplastic elastomer is thermoplastic polyurethane (TPU).

10. The multilayer breathing film-based aroma capsule (12) as claimed in claim 9, wherein said heat-transfer container (120) is selected from the material group consisting of plastic, polyester plastic, poly chloro terephthalate glycol (PCTG), polyethylene terephthalate (PET), propylthiouracil (PTU) and polypropylene (PP).

11. The multilayer breathing film-based aroma capsule (12) as claimed in claim 10, wherein said heat-transfer container (120) comprises a rim (1202) extended around said top opening (1200); said breathing film (126) is bonded to said rim (1202) of said heat-transfer container (120) around said top opening (1200).

12. The multilayer breathing film-based aroma capsule (12) as claimed in claim 11, wherein said heat-transfer container (120) further comprises a sealing cover bonded to said top opening (1200), said sealing cover comprising an inner protruding portion (1241) protruded from an inner surface thereof detachable fastened to said rim (1202) of said heat-transfer container (120) around said top opening (1200).

13. The multilayer breathing film-based aroma capsule (12) as claimed in claim 7, wherein said heat-transfer container (120) comprises a bottom recess (1204) located on a bottom wall thereof and curved upwards.

14. The multilayer breathing film-based aroma capsule (12) as claimed in claim 7, wherein the breathable aperture of said first pores (1263) is greater than the breathable aperture of said second pores (1264).

15. The multilayer breathing film-based aroma capsule (12) as claimed in claim 7, wherein the porosity of said first pores (1263) and the porosity of said second pores (1264) are different.
